(19) Europäisches Patentamt
European Patent Office
Office européen des brevets

(11) Veröffentlichungsnummer: 0 514 710 A2

(12) EUROPÄISCHE PATENTANMELDUNG

(21) Anmeldenummer: 92107672.5

(51) Int. Cl.5: C07C 213/06, C07D 211/46

(22) Anmeldetag: 07.05.92

(30) Priorität: 23.05.91 DE 4116818

(43) Veröffentlichungstag der Anmeldung: 25.11.92 Patentblatt 92/48

(84) Benannte Vertragsstaaten: BE CH DE FR GB LI

(71) Anmelder: BASF Aktiengesellschaft
Carl-Bosch-Strasse 38
W-6700 Ludwigshafen(DE)

(72) Erfinder: Karcher, Michael, Dr.
Helmholtzstrasse 20
W-6830 Schwetzingen(DE)
Erfinder: Henkelmann, Jochem, Dr.
Merianstrasse 5
W-6700 Ludwigshafen(DE)

# (54) Verfahren zur Herstellung von Aminovinylethern.

(57) Verfahren zur Herstellung von Aminovinylethern der allgemeinen Formel I

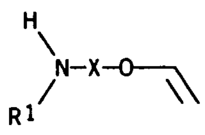

(I),

in der

X $C_3$- bis $C_{20}$-Alkyl, $C_3$- bis $C_8$-Cycloalkyl, Aryl oder $(CH_2)_nCHR^2$,

$R^1$ Wasserstoff, $C_1$- bis $C_{20}$-Alkyl, $C_3$- bis $C_8$-Cycloalkyl, Aryl oder gemeinsam mit $R^2$ eine $C_1$- bis $C_7$-Alkylenkette und

n eine ganze Zahl von 1 bis 4

bedeuten, indem man einen Aminoalkohol der allgemeinen Formel II

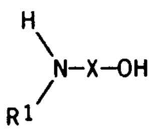

(II),

in der X, $R^1$, $R^2$ und n die oben genannten Bedeutungen haben, mit Acetylen in einem Gemisch aus Dimethylsulfoxid und einer Base bei Temperaturen von 60 bis 160 °C und bei Drücken von 1 bis 25 bar umsetzt.

EP 0 514 710 A2

Die vorliegende Erfindung betrifft ein Verfahren zur Herstellung von Aminovinylethern mit primären oder sekundären Aminogruppen durch Umsetzung von Aminoalkoholen mit Acetylen in Gemischen von Dimethylsulfoxid und einer Base bei erhöhten Temperaturen und Drücken von 1 bis 25 bar.

Aus M.F. Shostakovsky et al., Izvest. Akad. Mauk. S.S.S.R., Otdel. Khim. Nauk 971 bis 976 (1954) ist die basenkatalysierte Addition von Acetylen an den entsprechenden Alkohol (Reppe-Vinylierung) bekannt, die nur mit mäßigen Ausbeuten gelingt. Es bilden sich größere Mengen verharzter Produkte, die im Destillationssumpf zurückbleiben. Die auftretenden Schwierigkeiten sind bei der Vinylierung von Alkoholen mit primären Aminogruppen größer als mit sekundären Aminogruppen.

Von B.A. Trofimov, Uspekhi Khimii 50, 248 bis 272 (1981) wurden erstmals sogenannte Superbasen wie Dimethylsulfoxid/Kaliumhydroxid als Katalysatoren für Vinylierungsreaktionen eingesetzt. Durch Verwendung dieses Katalysators konnten Vinylyerbindungen z.B. von ungesättigten Alkoholen wie Allylalkohol (Trofimov, B.A. et al, Zh. Org. Khim (1990), 26(4), 725, die durch klassische Reppe-Vinylierung nicht zugänglich sind, hergestellt werden.

Ferner ist aus Trofimov, B.A., Zh. Prikl. Khim. (1990), 63(4), 835 eine verbesserte Synthese von 2-Vinyloxyethylamin mit Natriumhydroxid als Katalysator bei Normaldruck und Reaktionstemperaturen von 120 bis 150°C bekannt. In dieser Arbeit wurde erstmals auch die Tauglichkeit des DMSO/KOH-Katalysators zur Vinylierung eines Aminoalkohols geprüft. Es zeigte sich jedoch, daß mit diesem Katalysator die Ausbeute an dem gewünschten Vinylether nur mäßig war, da sich das Produkt teilweise zersetzte.

Der vorliegenden Erfindung lag daher die Aufgabe zugrunde, den zuvor genannten Nachteilen abzuhelfen.

Demgemäß wurde ein neues und verbessertes Verfahren zur Herstellung von Aminovinylethern der allgemeinen Formel I

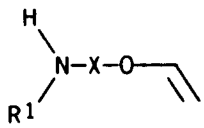

(I),

in der

X $C_3$- bis $C_{20}$-Alkyl, $C_3$- bis $C_8$-Cycloalkyl, Aryl oder $(CH_2)_nCHR^2$,

$R^1$ Wasserstoff, $C_1$- bis $C_{20}$-Alkyl, $C_3$- bis $C_8$-Cycloalkyl, Aryl oder gemeinsam mit $R^2$ eine $C_1$- bis $C_7$-Alkylenkette und

n eine ganze Zahl von 1 bis 4

bedeuten, gefunden, welches dadurch gekennzeichnet ist, daß man einen Aminoalkohol der allgemeinen Formel II

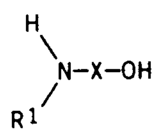

(II),

in der X, $R^1$, $R^2$ und n die oben genannten Bedeutungen haben, mit Acetylen in einem Gemisch aus Dimethylsulfoxid und einer Base bei Temperaturen von 60 bis 160 °C und bei Drücken von 1 bis 25 bar umsetzt.

Das erfindungsgemäße Verfahren läßt sich wie folgt durchführen:

Man legt den Aminoalkohol II, Dimethylsulfoxid und die Base in einem Autoklaven vor, gibt gasförmig Acetylen zu und läßt die Reaktion bei Temperaturen von 60 bis 160 °C, bevorzugt bei 90 bis 120 °C und Drücken von 1 bis 25 bar, vorzugsweise bei 2 bis 15 bar ablaufen. Aus Sicherheitsgründen kann man das eingesetzte Acetylen mit einem Inertgas wie Stickstoff oder Helium, Methan, Propan oder Argon, bevorzugt Stickstoff, verdünnen.

Die Aminoalkohole II sind z.B. aus Ullmanns Enzyklopädie der technischen Chemie, 4. Auflage, Band 8, 141 und Band 19, 433 bekannt oder lassen sich nach bekannten Methoden (Houben-Weyl, Methoden der Org. Chemie, 4. Auflage, 6/1a/1, 412 und 11/1, 11/2) herstellen.

Als Basen eignen sich Alkalihydroxide wie Lithiumhydroxid, Natriumhydroxid, Kaliumhydroxid, Rubidiumhydroxid und Caesiumhydroxid, bevorzugt Natriumhydroxid, Kaliumhydroxid und Caesiumhydroxid, besonders bevorzugt Kaliumhydroxid oder Alkalialkoholate z.B. diejenigen von $C_1$- bis $C_8$-Alkoholen wie

Methanol, Ethanol, Isopropanol, tert.-Butanol, Pentanol, Cyclohexanol bevorzugt. Methanol, Ethanol, tert.-Butanol, besonders bevorzugt Methanol und tert.-Butanol.

Als Basen besonders geeignet sind die Alkalialkoholate der entsprechenden Aminoalkohole II. Sie können entweder durch Reaktion der Aminoalkohole mit einem Alkalimetall oder durch andestillieren Alkalihydroxyd oder Alkalialkoholat enthaltender Lösungen (Abdestillieren von Wasser bzw. dem entsprechenden Alkohol des eingesetzten Alkalialkoholats) erhalten werden.

Die Vinylierung kann sowohl diskontinuierlich als auch kontinuierlich durchgeführt werden. Als Reaktionsgefäße eigenen sich sowohl gerührte Autoklaven als auch stehende Rohrreaktoren. Das Verfahren kann sowohl mit Gasphase als auch gasphasenfrei durchgeführt werden.

Man setzt bei der Reaktion Acetylen zum Aminoalkohol II im Molverhältnis von 1:1 bis 100:1, vorzugsweise 1,2:1 bis 30:1, besonders bevorzugt von 1,5:1 bis 10:1 ein.

Das Molverhältnis der eingesetzten Base zum Aminoalkohol II sollte zwischen 0,1 und 50 Mol.-%, bevorzugt zwischen 3 und 30 Mol.-%, besonders bevorzugt zwischen 5 und 20 Mol.-% liegen.

Die Aufarbeitung kann nach üblichen Methoden durchgeführt werden und erfolgt im allgemeinen durch einfache Destillation des rohen Vinylierungsproduktes. Haben Vinylether und Dimethylsulfoxid zu ähnliche Siedepunkte, so wird das Rohprodukt wäßrig aufgearbeitet.

Das Zwischenglied X, die Substituenten $R^1$, $R^2$ und der Index n in den Verbindungen I und II haben folgende Bedeutungen:

X

- unverzweigtes oder verzweigtes $C_3$- bis $C_{20}$-Alkyl, vorzugsweise unverzweigtes oder verzweigtes $C_3$- bis $C_8$-Alkyl, wie n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl,
- $C_3$- bis $C_8$-Cycloalkyl, wie Cyclopropyl, Cyclobutyl, Cyclopentyl, Cyclohexyl, Cycloheptyl und Cyclooctyl,
- Aryl, wie Phenyl, 1-Naphthyl und 2-Naphthyl, bevorzugt Phenyl,
- $(CH_2)_nCHR^2$

$R^1$

- Wasserstoff
- unverzweigtes oder verzweigtes $C_1$- bis $C_{20}$-Alkyl, vorzugsweise unverzweigtes oder verzweigtes $C_1$- bis $C_8$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl,
- $C_3$- bis $C_8$-Cycloalkyl, wie Cyclopropyl, Cyclobutyl, Cyclopentyl, Cyclohexyl, Cycloheptyl und Cyclooctyl,
- Aryl, wie Phenyl, 1-Naphthyl und 2-Naphthyl, bevorzugt Phenyl,
- gemeinsam mit $R^2$ eine $C_1$- bis $C_7$-Alkylenkette wie $-CH_2-$, $-(CH_2)_2-$, $-(CH_2)_3-$, $-(CH_2)_4-$, $-(CH_2)_5-$, $-(CH_2)_6-$ und $-(CH_2)_7-$, bevorzugt $-(CH_2)_2-$, $-(CH_2)_3-$ und $-(CH_2)_4-$, besonders bevorzugt $-(CH_2)_2-$ und $-(CH_2)_3-$,

n

- eine ganze Zahl von 1 bis 4 wie 1, 2, 3 und 4, bevorzugt 2 und 3.

Bevorzugte Aminovinylether der allgemeinen Formel I sind:
3-Vinyloxy-1-propylamin, 2-Vinyloxy-1-propylamin, 4-Vinyloxypiperidin, 2,2'-Bis(vinyloxy)diethylamin.

Die Aminovinylether eignen sich als Monomere für Kunststoffe sowie als Zwischenprodukte.

## Beispiele

### Beispiel 1

Herstellung von 3-Vinyloxy-1-propylamin

30 g (0,4 mol) 3-Amino-1-propanol wurden in 120 g Dimethylsulfoxid gelöst, mit 3,75 g (0,067 mol) KOH versetzt und in einen 300-ml-Hochdruckautoklaven eingefüllt. Der Autoklav wurde mit Stickstoff gespült und auf 90°C erhitzt. Zuerst werden 10 bar Stickstoff und danach 10 bar Acetylen aufgepreßt. In dem Maße, in dem das Acetylen verbraucht wurde, wurde wieder frisches Acetylen zugegeben. Nach Reaktionsende (Acetylenaufnahme 0,2 bar/h) wird das rohe Vinylierungsgemisch destilliert. Man erhielt 38 g (93 %) 3-Vinyloxy-1-propylamin; Sdp: 40°C/7 mbar.

Beispiel 2

Herstellung von 3-Vinyloxy-1-propylamin

Analog Beispiel 1 wurden 75 g (1 mol) 3-Amino-1-propanol, 120 g Dimethylsulfoxid, 3,75 g (0,067 mol) KOH eingesetzt. Man erhielt 95 g (94 %) 3-Vinyloxy-1-propylamin.

Beispiel 3

Herstellung von 5-Vinyloxy-1-pentylamin

Analog Beispiel 1 wurden 30 g (0,29 mol) 5-Amino-1-pentanol, 120 g Dimethylsulfoxid, 3,75 g (0,067 mol) KOH eingesetzt. Man erhielt 36 g (96 %) 5-Vinyloxy-1-pentylamin; Sdp. 52 °C/7 mbar.

Beispiel 4

Herstellung von 2-Vinyloxy-1-propylamin

Analog Beispiel 1 wurden 30 g (0,4 mol) 1-Amino-2-propanol, 120 g Dimethylsulfoxid, 3,75 g (0,067 mol) KOH eingesetzt. Man erhielt 38 g (94 %) 2-Vinyloxy-1-propylamin; Sdp. 35°C/10 mbar.

Beispiel 5

Herstellung von 1-Vinyloxy-2-butylamin

Analog Beispiel 1 wurden 30 g (0,34 mol) 2-Amino-1-butanol, 120 g Dimethylsulfoxid, 3,75 g (0,067 mol) KOH eingesetzt. Man erhielt 36 g (93 %) 1-Vinyloxy-2-butylamin; Sdp. 45°C/28 mbar.

Beispiel 6

Herstellung von N-Ethyl-2-vinyloxy-1-ethylamin

Analog Beispiel 1 wurden 30 g (0,34 mol) 2-N-Ethyl-2-amino-1-ethanol, 120 g Dimethylsulfoxid, 7,5 g (0,134 mol) KOH eingesetzt. Man erhielt 38 g (98 %) N-Ethyl-2-vinyloxy-1-ethylamin; Sdp. 32°C/25 mbar.

Beispiel 7

Herstellung von N-Butyl-2-vinyloxy-1-ethylamin

Analog Beispiel 1 wurden 30 g (0,26 mol) 2-N-Butyl-2-amino-1-ethanol, 120 g Dimethylsulfoxid, 3,75 g (0,067 mol) KOH eingesetzt. Man erhielt 31 g (84 %) N-Butyl-2-vinyloxy-1-ethylamin; Sdp.30°C/8 mbar.

Beispiel 8

Herstellung von 4-Vinyloxypiperidin

Analog Beispiel 1 wurden 30 g (0,3 mol) 4-Hydroxypiperidin, 120 g Dimethylsulfoxid, 3,75 g (0,067 mol) KOH eingesetzt. Man erhielt 36 g (95 %) 4-Vinyloxypiperidin; Sdp. 60°C/12 mbar.

Beispiel 9

Herstellung von N-Methyl-2-vinyloxy-1-ethylamin

Analog Beispiel 1 wurden 30 g (0,4 mol) 2-N-Methyl-2-amino-1-ethanol, 120 g Dimethylsulfoxid, 7,5 g (0,134 mol) KOH eingesetzt. Man erhielt 40 g (98 %) N-Methyl-2-vinyloxy-1-ethylamin; Sdp. 28°C/30 mbar.

Beispiel 10

Herstellung von N-Cyclohexyl-2-vinyloxy-1-ethylamin

Analog Beispiel 1 wurden 30 g (0,2 mol) 2-N-Cyclohexyl-2-amino-1-ethanol,120 g Dimethylsulfoxid, 3,75 g (0,067 mol) KOH eingesetzt. Man erhielt 34 g (96 %) N-Cyclohexyl-2-vinyloxy-1-ethylamin; Sdp. 46°C/1,1 mbar.

Beispiel 11

Herstellung von 2,2'-Bis(vinyloxy)diethylamin

In einem 20 1-Autoklaven wurden analog Beispiel 1 2280 g (21,71 mol) Diethanolamin, 9720 g Dimethylsulfoxid, 570 g (10,17 mol) KOH eingesetzt. Man erhielt 3176 g (93 %), 2,2'-Bis(vinyloxy)-diethylamin; Sdp. 47°C/1 mbar.

Beispiel 12

Herstellung von N-(2-Aminoethyl)-2-amin-1-ethanol

Analog Beispiel 1 wurden 30 g (0,29 mol) N-(2-Vinyloxyethyl)-2-amino-1-ethylamin, 120 g Dimethylsulfoxid, 3,75 g (0,067 mol) KOH eingesetzt. Man erhielt 34 g (90 %) N-(2-Aminoethyl)-2-amino-1-ethanol; Sdp. 70°C/5 mbar.

## Patentansprüche

**1.** Verfahren zur Herstellung von Aminovinylethern der allgemeinen Formel I

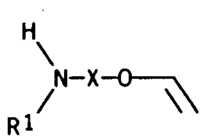

(I),

in der

- X $C_3$- bis $C_{20}$-Alkyl, $C_3$- bis $C_8$-Cycloalkyl, Aryl oder $(CH_2)_nCHR^2$,
- $R^1$ Wasserstoff, $C_1$- bis $C_{20}$-Alkyl, $C_3$- bis $C_8$-Cycloalkyl, Aryl oder gemeinsam mit $R^2$ eine $C_1$- bis $C_7$-Alkylenkette und
- n eine ganze Zahl von 1 bis 4

bedeuten, dadurch gekennzeichnet, daß man einen Aminoalkohol der allgemeinen Formel II

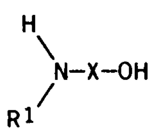

(II),

in der X, $R^1$, $R^2$ und n die oben genannten Bedeutungen haben, mit Acetylen in einem Gemisch aus Dimethylsulfoxid und einer Base bei Temperaturen von 60 bis 160 °C und bei Drücken von 1 bis 25 bar umsetzt.

**2.** Verfahren zur Herstellung von Aminovinylethern I nach Anspruch 1, dadurch gekennzeichnet, daß man als Base ein Alkalihydroxid oder ein Alkalialkoholat verwendet.

**3.** Verfahren zur Herstellung von Aminovinylethern I nach Anspruch 1, dadurch gekennzeichnet, daß man als Base Kaliumhydroxid verwendet.

**4.** Verfahren zur Herstellung von Aminovinylethern I nach Anspruch 1, dadurch gekennzeichnet, daß man

als Base Kalium-tert.-butylat verwendet.

5. Verfahren zur Herstellung von Aminovinylethern I nach Anspruch 1, dadurch gekennzeichnet, daß man als Base die Kaliumverbindung des entsprechenden Aminoalkohols II einsetzt.

6. Verfahren zur Herstellung von Aminovinylethern I nach Anspruch 1, dadurch gekennzeichnet, daß man Acetylen zum Aminoalkohol II im Molverhältnis von 1:1 bis 100:1 verwendet.

7. Verfahren zur Herstellung von Aminovinylethern I nach Anspruch 1, dadurch gekennzeichnet, daß man 0,1 bis 50 Mol.-% Base bezogen auf den eingesetzten Aminoalkohol II verwendet.